# EUROPEAN PATENT APPLICATION

(11) **EP 4 166 575 A1**
(43) Date of publication of application: **19.04.2023**
(21) Application number: 21823037.3
(22) Date of filing: 11.06.2021
(51) Int. Cl.: C07K 19/00, C07K 1/06, C07K 1/20, C07K 1/16, C12N 15/70, C12P 21/06, C12N 15/62, C12N 1/21, A61K 38/26, A61P 3/10

(54) **SEMAGLUTIDE DERIVATIVE, AND PREPARATION METHOD THEREFOR AND APPLICATION THEREOF**

(30) Priority: 11.06.2020 CN 202010531568; 24.07.2020 CN 202010724452
(71) Applicant: Ningbo Kunpeng Biotech Co., Ltd., Yuyao Ningbo, Zhejiang 315400 (CN)
(72) Inventor: YU, Ge, Ningbo, Zhejiang 315400 (CN); LIU, Huiling, Ningbo, Zhejiang 315400 (CN); CHEN, Wei, Ningbo, Zhejiang 315400 (CN)
(74) Representative: Lavoix
(86) International application number: PCT/CN2021/099877
(87) International publication number: WO 2021/249564

(57) **Abstract**

Provided are a semaglutide derivative and a preparation method therefor. Specifically, provided is a fusion protein comprising a green fluorescent protein folding unit and a semaglutide precursor or an active fragment thereof. The expression level of the fusion protein is significantly improved. Moreover, the green fluorescent protein folding unit in the fusion protein can be digested into small fragments by a protease; and compared with a target protein, a molecular weight difference is large, and the fusion protein is easily separated. Further provided are a method for preparing semaglutide by using the fusion protein and a method for preparing an intermediate.

## Description

### TECHNICAL FIELD

The present invention relates to the field of biomedicine, in particular to an semaglutide derivative and application thereof.

### BACKGROUND

Diabetes is a major disease that threatens human health worldwide. In China, with the change of people's lifestyle and the acceleration of aging, the prevalence of diabetes is increasing rapidly. Acute and chronic complications of diabetes, especially the chronic complications, involve multiple organs, cause high disability and mortality rates, seriously affect the physical and mental health of patients, and bring heavy burdens to individuals, families and society.

Semaglutide is an antidiabetic drug developed by Novo Nordisk, which can significantly reduce glycated hemoglobin (HbA1c) levels and reduce weight in patients with type 2 diabetes and greatly reduce the risk of hypoglycemia. Semaglutide is obtained by modifying GLP-1 (7-37). Compared with Liraglutide, Semaglutide has longer fat chains and increased hydrophobicity. However, the hydrophilicity of Semaglutide is greatly enhanced by PEG modification of its short chains. PEG modification can not only make it bind tightly to albumin and mask the DPP-4 enzymatic hydrolysis site, but also reduce renal excretion, prolong the biological half-life, and achieve the effect of long circulation.

The CAS number of semaglutide is 910463-68-2, the English name thereof is Semaglutide, and its sequence is as follows: H-His1-Aib2-Glu3-Gly4-Thr5-Phe6-Thr7-Ser8-Asp9-Val10-Ser11-Ser12-Tyr13-Le u14-Glu15-Gly16-Gln17-Ala18-Ala19-Lys20(PEG2-PEG2-γ-Glu-Octadecanedioic acid)-Glu21-Phe22-Ile23-Ala24-Trp25-Leu26-Val27-Arg28-Gly29-Arg30-Gly31-O H.

In the Patent Application No. CN201611095162, fully protected semaglutide is synthesized and obtained by fragment condensation method, and cleavaged to obtain semaglutide crude peptide. Since this method employs fragments to condensation, its raw materials are not readily available, and are costly. In addition, the main chain is firstly condensed to the Thr at position 5, and then the side chain protection group Alloc on the Lys at position 20 is removed to condense the side chain. This method is easy to cause polycondensation of fragment 2 resin during the synthesis process, which greatly reduces the coupling efficiency of amino acids after Lys at position 20 and fragment 1, and is easy to generate racemic impurities, which is disadvantageous for industrial production.

In the Patent Application No. CN201511027176, fully protected semaglutide resin is obtained via solid-phase synthesis method, and cleavaged to obtain semaglutide crude peptide, which is purified to obtain pure semaglutide. In this method, the main chain is firstly condensed, and then the side chain protection group Alloc on the Lys is removed to condense the side chain. This method is easy to cause polycondensation of resin during the synthesis process, which greatly reduces the coupling efficiency, and is easy to generate racemic impurities, especially the racemic of the last amino acid His, which greatly reduces the yield of the product and increases the cost of production.

Therefore, those skilled in the art are committed to new methods for producing semaglutide.

### SUMMARY OF THE INVENTION

The purpose of the present invention is to provide a semaglutide derivative and application thereof.

In the first aspect of the present invention, it provides a semaglutide precursor fusion protein having the structure as shown in Formula I from N-terminus to the C-terminus:

A-FP-TEV-EK-G (I)

wherein,
"-" represents a peptide bond;
A is absent or a leader peptide sequence,
FP is a green fluorescent protein folding unit;
TEV is the first restriction site, and preferably is a restriction site of TEV enzyme (as shown in sequence ENLYFQG, SEQ ID NO: 8);
EK is the second restriction site, and preferably is a restriction site of enterokinase (as shown in sequence DDDDK, SEQ ID NO: 9);
G is a semaglutide precursor or a fragment thereof;
wherein the green fluorescent protein folding unit comprises 2-6 β-folding units selected from the group consisting of:

| β-folding unit | | Amino acid sequence |
|---|---|---|
| | u1 | VPILVELDGDVNG (SEQ ID NO: 11) |
| | u2 | HKFSVRGEGEGDAT (SEQ ID NO: 12) |
| | u3 | KLTLKFICTT (SEQ ID NO: 13) |
| | u4 | YVQERTISFKD (SEQ ID NO: 14) |
| | u5 | TYKTRAEVKFEGD (SEQ ID NO: 15) |
| | u6 | TLVNRIELKGIDF (SEQ ID NO: 16) |
| | u7 | HNVYITADKQ (SEQ ID NO: 17) |
| | u8 | GIKANFKIRHNVED (SEQ ID NO: 18) |
| | u9 | VQLADHYQQNTPIG (SEQ ID NO: 19) |
| | u10 | HYLSTQSVLSKD (SEQ ID NO: 20) |
| | u11 | HMVLLEFVTAAGI (SEQ ID NO: 21). |

In another preferred embodiment, the green fluorescent protein folding unit is u2-u3, u4-u5, u1-u2-u3, u3-u4-u5 or u4-u5-u6.

In another preferred embodiment, the G is a Boc-modified semaglutide precursor which lacks 2-7 amino acids of the N-terminus of the semaglutide main chain, and the lysine contained therein is modified with Boc.

In another preferred embodiment, the ε-amino of the Boc-modified lysine is modified with tert-butoxycarbonyl.

In another preferred embodiment, the amino acid sequence of the semaglutide main chain is as shown in SEQ ID NO: 3.

In another preferred embodiment, the semaglutide precursor comprises:
a first semaglutide precursor modified with Boc at position 18, whose amino acid sequence is as shown in SEQ ID NO: 1;
or, a second semaglutide precursor modified with Boc at position 17, whose amino acid sequence is as shown in SEQ ID NO: 2;
or, a third semaglutide precursor modified with Boc at position 16, whose amino acid sequence is as shown in SEQ ID NO: 23;
or, a fourth semaglutide precursor modified with Boc at position 15, whose amino acid sequence is as shown in SEQ ID NO: 24;
or, a fifth semaglutide precursor modified with Boc at position 14, whose amino acid sequence is as shown in SEQ ID NO: 25.
SEQ ID NO: 1: EGTFTSDVSSYLEGQAA**K**EFIAWLVRGRG
SEQ ID NO: 2: GTFTSDVSSYLEGQAA**K**EFIAWLVRGRG
SEQ ID NO: 23: TFTSDVSSYLEGQAA**K**EFIAWLVRGRG
SEQ ID NO: 24: FTSDVSSYLEGQAA**K**EFIAWLVRGRG
SEQ ID NO: 25: TSDVSSYLEGQAA**K**EFIAWLVRGRG
(the underlined K is the Boc-modified lysine).

In another preferred embodiment, the amino acid at position 4 of C-terminus of the semaglutide precursor is an arginine or lysine.

In another preferred embodiment, the arginine at position 4 of C-terminus of the semaglutide precursor may be substituted with a lysine.

In another preferred embodiment, the amino acid at position 4 of C-terminus of the fusion protein is an arginine or lysine.

In another preferred embodiment, the arginine at position 4 of C-terminus of the fusion protein can be substituted with a lysine.

In the present application, the complete semaglutide sequence (H(Aib)EG TFTSDVSSYLEGQAA**K**EFIAWLVRGRG, SEQ ID NO: 3) is defined as the main chain of semaglutide, and the semaglutide lacking N-terminal amino aci d is defined as the semaglutide precursor. For Fmoc-modified semaglutide m ain chain, the H at its N-terminus is modified by Fmoc. For the Boc-modifi ed semaglutide main chain, the lysine at position 20 is a Nε-(tert-butoxycarb onyl)-lysine.

In another preferred embodiment, the green fluorescent protein folding unit is u3-u4-u5.

In another preferred embodiment, the amino acid sequence of the leader peptide is as shown in SEQ ID NO: 7.

In another preferred embodiment, the 14th, 15th, 16th, 17th or 18th position of the semaglutide precursor is a Nε-(tert-butoxycarbonyl)-lysine (i.e., in each semaglutide precursor, the amino acid corresponding to the 20th position of the semaglutide main chain is a Nε-(tert-butoxycarbonyl)-lysine).

In the second aspect of the present invention, it provides a Fmoc and Boc-modified semaglutide main chain, wherein the position 20 of the semaglutide main chain is a protected lysine, which is a Nε- (tert-butoxycarbonyl)-lysine, and the N-terminus of the semaglutide main chain is a Fmoc-modified histidine.

In another preferred embodiment, the Fmoc is a fluorenylmethoxycarbonyl.

In another preferred embodiment, the amino acid sequence of the semaglutide main chain is as shown in SEQ ID NO: 3.

In the third aspect of the present invention, it provides a Boc-modified semaglutide precursor which comprises:
a first semaglutide precursor modified with Boc at position 18, whose amino acid sequence is as shown in SEQ ID NO: 1;
or, a second semaglutide precursor modified with Boc at position 17, whose amino acid sequence is as shown in SEQ ID NO: 2;
or, a third semaglutide precursor modified with Boc at position 16, whose amino acid sequence is as shown in SEQ ID NO: 23;
or, a fourth semaglutide precursor modified with Boc at position 15, whose amino acid sequence is as shown in SEQ ID NO: 24;
or, a fifth semaglutide precursor modified with Boc at position 14, whose amino acid sequence is as shown in SEQ ID NO: 25.

In the fourth aspect of the present invention, it provides a Fmoc-modified semaglutide main chain, wherein the N-terminus of the semaglutide main chain is a Fmoc-modified histidine, and the amino acid sequence of the semaglutide main chain is shown in SEQ ID NO: 3.

In the fifth aspect of the present invention, it provides a method for preparing a semaglutide, which comprises the steps:
(A) using recombinant bacteria to ferment, to prepare a semaglutide precursor fusion protein, and
(B) using the semaglutide precursor fusion protein to prepare the semaglutide,
   wherein the semaglutide precursor fusion protein is as described in the first aspect of the present invention.

In another preferred embodiment, the step (B) further comprises the steps:
(i) digesting the semaglutide precursor fusion protein, thereby obtaining a Boc-modified semaglutide precursor that lacks X amino acids at the N-terminus of the semaglutide main chain, wherein X is an integer of 2-7;
(ii) conjugating a Fmoc complex to the N-terminus of the Boc-modified semaglutide precursor, thereby obtaining a Fmoc and Boc-modified semaglutide main chain;
   wherein the Fmoc complex comprises X amino acids at the N-terminus of the semaglutide main chain, and the N-terminal amino acids of the Fmoc complex are modified with Fmoc;
(iii) removing the Boc from the Fmoc and Boc-modified semaglutide main chain, and reacting the same with a semaglutide side chain, thereby obtaining a Fmoc-modified semaglutide; and
(iv) removing the Fmoc from the Fmoc-modified semaglutide, thereby obtaining a Fmoc-removed semaglutide;
(v) removing the OtBu from the side chain of the Fmoc-removed semaglutide, thereby obtaining the semaglutide.

In another preferred embodiment, in step (i), enterokinase is used for the digestion.

In another preferred embodiment, the Boc-modified semaglutide precursor comprises:
a first semaglutide precursor modified with Boc at position 18, whose amino acid sequence is shown in SEQ ID NO: 1;
or, a second semaglutide precursor modified with Boc at position 17, whose amino acid sequence is shown in SEQ ID NO: 2;
or, a third semaglutide precursor modified with Boc at position 16, whose amino acid sequence is shown in SEQ ID NO: 23;
or, a fourth semaglutide precursor modified with Boc at position 15, whose amino acid sequence is shown in SEQ ID NO: 24;
or, a fifth semaglutide precursor modified with Boc at position 14, whose amino acid sequence is ahown in SEQ ID NO: 25.

In another preferred embodiment, the Fmoc complex is Fmoc-H-Aib, Fmoc-H-Aib-E, Fmoc-H-Aib-E-G-T-F, Fmoc-H-Aib-E-G-T or Fmoc-H-Aib-E-G.

In another preferred embodiment, in steps (i) and (ii), the values of X are the same.

In another preferred embodiment, the Fmoc and Boc-modified semaglutide main chain is as described in the second aspect of the present invention.

In another preferred embodiment, the reaction of step (ii) is as follow: 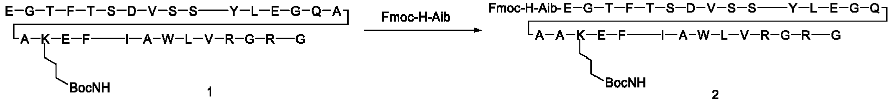 or 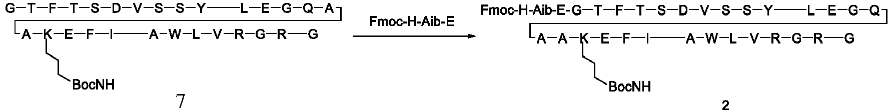 or 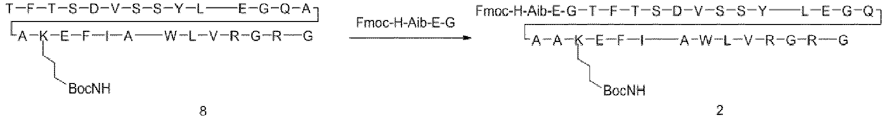 or 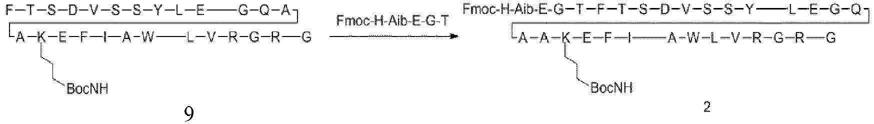 or

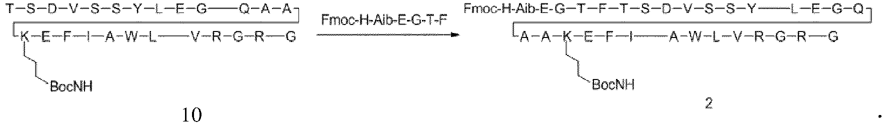

In another preferred embodiment, the semaglutide side chain is as follow:

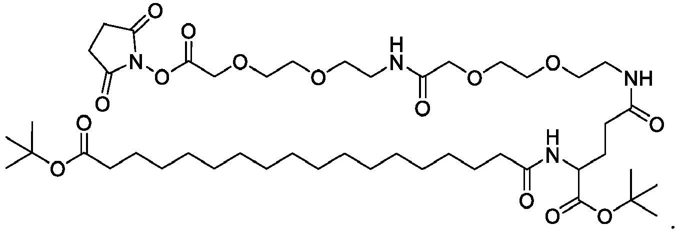

In another preferred embodiment, in step (ii), Fmoc complex (activated ester), DIPEA (N, N-diisopropylethylamine) and DMF (N, N-dimethylformamide) are added to conjugate the Fmoc complex to the N-terminus of the Boc-modified semaglutide precursor.

In another preferred embodiment, the Fmoc complex is an Fmoc complex in the form of an activated ester, which is formed by activation with HOSu/DCC, HoBt/DIC, and TBTU/DIPEA.

In another preferred embodiment, the molar ratio of the added Fmoc complex (activated ester), DIPEA and Boc-modified semaglutide precursor is (1.0-3.0) : (10-14) : (0.8-1.2), and preferably (2-2.8) : (11-13) : (0.8-1.2).

In another preferred embodiment, it further comprises a step of purification of the obtained Fmoc and Boc-modified semaglutide main chain between steps (ii) and (iii).

In another preferred embodiment, the purification is to add an organic solvent, preferably a mixture of methyl tert-butyl ether/petroleum ether, to the reaction solution, thereby obtaining a solid product.

In another preferred embodiment, in step (iii), it further comprises the steps:
(a) adding the Compound 2 (Fmoc and Boc-modified semaglutide main chain) to pre-cooled TFA solution at 0±5°C, stirring to remove Boc and obtaining the Boc-removed product;
(b) adding an organic solvent, preferably a mixture of methyl tert-butyl ether/petroleum ether, to the reaction solution of step (a), thereby obtaining a Boc-removed solid product;
(c) mixing the Boc-removed product with the semaglutide side chain to obtain the Fmoc-modified semaglutide.

In another preferred embodiment, in step (c), the Boc-removed solid product is mixed with the semaglutide side chain in DMF and reacted at room temperature.

In another preferred embodiment, in step (c), the reaction system further comprises DIPEA.

In another preferred embodiment, in step (iv), DMF solution containing piperidine is added to remove Fmoc, thereby obtaining the Fmoc-removed semaglutide.

In another preferred embodiment, in step (v), a mixture solution of TFA, TIS and DCM is added to remove the OtBu protection group from the side chain, thereby obtaining the semaglutide.

In another preferred embodiment, in step (v), it further comprises a step of purification of the obtained semaglutide.

In another preferred embodiment, the Boc-modified semaglutide precursor is produced by using genetic recombination technique.

In another preferred embodiment, in step (A), inclusion bodies of the semaglutide precursor fusion protein are isolated and obtained from the fermentation broth of the recombinant bacteria, renatured and digested to obtain the semaglutide precursor fusion protein.

In another preferred embodiment, it further comprises a purification step, preferably a reverse phase chromatography, before and after step (i).

In another preferred embodiment, the recombinant bacterium comprises or is integrated with an expression cassette expressing the semaglutide precursor fusion protein.

In another preferred embodiment, the method is as follows:

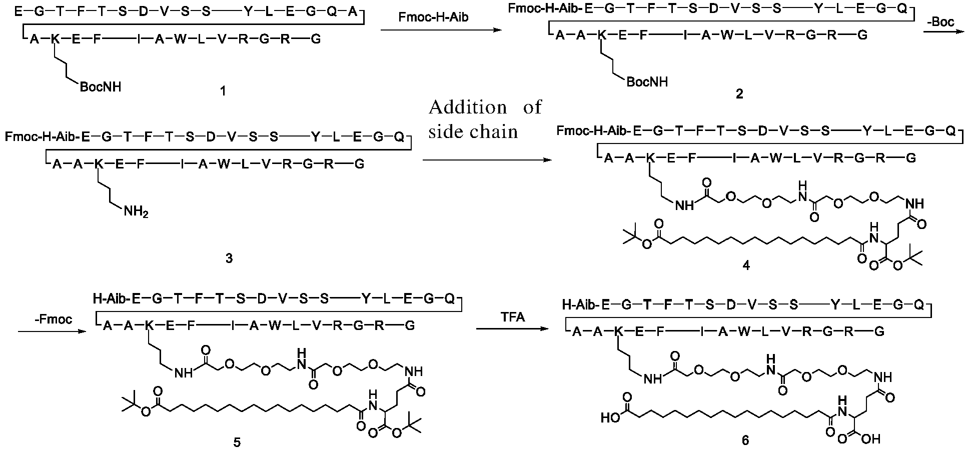

In another preferred embodiment, the method further comprises the steps:
(i) providing the semaglutide precursor fusion protein of the first aspect of the present invention to obtain a Compound 1 by enzyme digesting;
(ii) connecting the Compound 1 with an Fmoc-H-Aib complex, preferably in the form of an activated ester, thereby obtaining a Compound 2 (Fmoc and Boc-modified semaglutide main chain),
(iii) removing Boc from the Compound 2, and react the same with the semaglutide side chain, thereby obtaining a Compound 4; and
(iv) removing Fmoc from the Compound 4, thereby obtaining a Compound 5;
(v) removing the OtBu from the side chain of the Compound 5, thereby obtaining the semaglutide as shown in Compound 6.

In another preferred embodiment, the method further comprises the steps:
(i) providing the semaglutide precursor fusion protein of the first aspect of the present invention to obtain a Compound 7, Compound 8, Compound 9 or Compound 10 by enzyme digesting,
(ii) connecting the Compound 7 with a Fmoc-H-Aib-E complex, preferably in the form of an activated ester,
   or, connecting the Compound 8 with a Fmoc-H-Aib-E-G complex, preferably in the form of an activated ester,
   or, connecting the Compound 9 with a Fmoc-H-Aib-E-G-T complex, preferably in the form of an activated ester,
   or, connecting the Compound 10 with a Fmoc-H-Aib-E-G-TF complex, preferably in the form of an activated ester;
   thereby obtaining a Compound 2,
(iii) removing Boc from the Compound 2, and react the same with the side chain of semaglutide, thereby obtaining a Compound 4; and
(iv) removing Fmoc from the Compound 4, thereby obtaining a Compound 5;
(v) removing the OtBu from the side chain of the Compound 5, thereby obtaining the semaglutide as shown in Compound 6.

In the sixth aspect of the present invention, it provides an isolated polynucleotide encoding the semaglutide precursor fusion protein of the first aspect of the present invention, the Fmoc and Boc-modified semaglutide main chain of the second aspect of the present invention, the Boc-modified semaglutide precursor of the third aspect of the present invention, or the Fmoc-modified semaglutide main chain of the fourth aspect of the present invention.

In the seventh aspect of the present invention, it provides a vector comprising the polynucleotide of the sixth aspect of the present invention.

In another preferred embodiment, the vector is selected from the group consisting of DNA, RNA, a plasmid, a lentiviral vector, an adenoviral vector, a retroviral vector, a transposon, and a combination thereof.

In the eighth aspect of the present invention, it provides a host cell comprising the vector of the seventh aspect of the present invention or in which the chromosome is integrated with exogenous polynucleotide of the sixth aspect of the present invention.

In another preferred embodiment, the host cell is selected from *Escherichia coli., Bacillus subtilis.,* a yeast cell, an insect cell, a mammalian cell, or a combination thereof.

In the ninth aspect of the present invention, it provides a formulation comprising the semaglutide precursor fusion protein of the first aspect of the present invention, the Fmoc and Boc-modified semaglutide main chain of the second aspect of the present invention, the Boc-modified semaglutide precursor of the third aspect of the present invention, or the Fmoc-modified semaglutide main chain of the fourth aspect of the present invention.

In the tenth aspect of the present invention, it provides a semaglutide formulation produced by using the method of the fifth aspect of the present invention.

### DESCRIPTION OF THE FIGURES

Figure 1 shows a map of the plasmid pBAD-FP-TEV-EK-GLP-1(18).
Figure 2 shows a map of the plasmid pBAD-FP-TEV-EK-GLP-1(17).
Figure 3 shows a map of the plasmid pEvol-pylRs-pylT.
Figure 4 shows the SDS-PAGE electrophoregram of the Boc-semaglutide precursor fusion protein inclusion body.
Figure 5 shows the HPLC detection spectrogram of the Boc-semaglutide precursor 1.
Figure 6 shows the HPLC detection spectrogram of the Boc-semaglutide precursor 3.
Figure 7 shows a map of the plasmid pBAD-FP-TEV-EK-GLP-1(16).

### DETAILED DESCRIPTION

After extensive and intensive research, the inventors have discovered a new method and process for preparing a semaglutide product. Specifically, in the method, the Fmoc orthogonal protection method is used to perform the side chain addition step during the preparation of semaglutide, and the conditions for purification and synthesis during the preparation process are optimized. The method of the present invention does not require expensive solid-phase synthesis instruments, shortens the production cycle, has simple production process, and improves the purity and yield of the product. Moreover, the present invention further provides a novel precursor fusion protein (Formula I) and corresponding intermediates (i.e., the Fmoc and Boc-modified semaglutide main chain and the Fmoc-modified semaglutide main chain). Using the precursor fusion protein of the present invention, the intermediates can be efficiently produced. Using the intermediates of the present invention, on the one hand, the condensation reaction between the 20-position Lys and the side chain can be performed with high efficiency and mild conditions. On the other hand, the N-terminal Fmoc has a good protective effect, and the removal condition thereof is mild and does not cause the racemic of N-terminal His, which barely produce racemic impurities. Studies have shown that the use of the optimized precursor fusion proteins and optimized intermediates of the present invention can greatly increase the yield of semaglutide and reduce production costs. On this basis, the present invention has been completed.

### Semaglutide

Semaglutide is developed by Novo Nordisk, of which the English name is Semaglutide, and CAS number is 204656-20-2, and is an analogue of hu man glucagon-like peptide-1 (GLP-1). Its sequence is: H-His1-Aib2-Glu3-Gly 4-Thr5-Phe6-Thr7-Ser8-Asp9-Val10-Ser11-Ser12-Tyr13-Leu14-Glu15-Gly16-Gln1 7-Ala18-Ala19-Lys20(PEG2-PEG2-γ-Glu-Octadecanedioic acid)-Glu21-Phe22-Ile 23-Ala24-Trp25-Leu26-Val27-Arg28-Gly29-Arg30-Gly31-OH. Its sequence horn ology to human native GLP-1 is 97%.

Semaglutide is an antidiabetic drug developed by Novo Nordisk, which can significantly reduce glycated hemoglobin (HbA1c) levels and reduce weight in patients with type 2 diabetes and greatly reduce the risk of hypoglycemia. Semaglutide is obtained by modifying GLP-1 (7-37). Compared with Liraglutide, Semaglutide has longer fat chains and increased hydrophobicity. However, the hydrophilicity of Semaglutide is greatly enhanced by PEG modification of its short chains. PEG modification can not only make it bind tightly to albumin, mask the DPP-4 enzymatic hydrolysis site, but also reduce renal excretion, prolong the biological half-life, and achieve the effect of long circulation. It can significantly reduce the fasting or postprandial blood glucose of patients with type 2 diabetes so as to regulate blood glucose levels in the body, as well as reduce the weight of patients and the risk of death in patients with cardiovascular disease.

### The protein of the present invention

As used herein, the term "the protein of the present invention" includes the precursor fusion protein of the present invention and the corresponding intermediates, and specifically includes the semaglutide precursor fusion protein of the first aspect of the present invention, the Fmoc and Boc-modified semaglutide main chain of the second aspect of the present invention, the Boc-modified semaglutide precursor of the third aspect of the present invention, or the Fmoc-modified semaglutide main chain of the fourth aspect of the present invention.

As used herein, the term "the intermediates of the present invention" includes the Fmoc and Boc-modified semaglutide main chain of the second aspect of the present invention, the Boc-modified semaglutide precursor of the third aspect of the present invention, or the Fmoc-modified semaglutide main chain of the fourth aspect of the present invention.

### Fusion protein

As used herein, the term "the protein of the present invention" and "the precursor fusion protein of the present invention", and "the semaglutide precursor fusion protein of the present invention" are used interchangeably, and refer to the semaglutide precursor fusion protein having a structure of Formula I of the first aspect of the present invention.

In the present invention, using the green fluorescent protein folding unit, the present inventor constructs a semaglutide precursor fusion protein, as described in the first aspect of the present invention.

The green fluorescent protein folding unit contained in the fusion protein of the present invention comprises 2-6, preferably 2-3, β-folding units selected from the group consisting of:

| | | Amino acid sequence |
|---|---|---|
| | u1 | VPILVELDGDVNG (SEQ ID NO: 11) |
| | u2 | HKFSVRGEGEGDAT (SEQ ID NO: 12) |
| | u3 | KLTLKFICTT (SEQ ID NO: 13) |
| | u4 | YVQERTISFKD (SEQ ID NO: 14) |
| | u5 | TYKTRAEVKFEGD (SEQ ID NO: 15) |
| | u6 | TLVNRIELKGIDF (SEQ ID NO: 16) |
| | u7 | HNVYITADKQ (SEQ ID NO: 17) |
| | u8 | GIKANFKIRHNVED (SEQ ID NO: 18) |
| | u9 | VQLADHYQQNTPIG (SEQ ID NO: 19) |
| | u10 | HYLSTQSVLSKD (SEQ ID NO: 20) |
| | u11 | HMVLLEFVTAAGI (SEQ ID NO: 21). |

In another preferred embodiment, the green fluorescent protein folding unit FP may be selected from the group consisting of: u8, u9, u2-u3, u4-u5, u8-u9, u1-u2-u3, u2-u3-u4, u3-u4-u5, u5-u6-u7, u8-u9-u10, u9-u10-u11, u3-u5-u7, u3-u4-u6, u4-u7-u10, u6-u8-u10, u1-u2-u3-u4, u2-u3-u4-u5, u3-u4-u3-u4, u3-u5-u7-u9, u5-u6-u7-u8, u1-u3-u7-u9, u2-u2-u7-u8, u7-u2-u5-u11, u3-u4-u7-u10, u1-I-u2, u1-I-u5, u2-I-u4, u3-I-u8, u5-I-u6, and u10-I-u11.

In another preferred embodiment, the green fluorescent protein folding unit is u3-u4-u5 or u4-u5-u6.

As used herein, the term "fusion protein" also includes variant forms having the above-mentioned activities. These variant forms include (but are not limited to): 1-3 (usually 1-2, more preferably 1) amino acid deletions, insertions and/or substitutions, and one or several (usually 3 or less, preferably 2 or less, more preferably 1 or less) amino acids added or deleted at the C- terminus and/or N-terminus. For example, in this field, when substituted with amino acids with close or similar properties, the function of the protein is usually not changed. For another example, adding or deleting one or several amino acids at the C-terminus and/or N-terminus usually does not change the structure and function of the protein. In addition, the term also includes the polypeptide of the present invention in monomeric and multimeric forms. The term also includes linear and non-linear polypeptides (such as cyclic peptides).

The present invention also includes active fragments, derivatives and analogs of the above-mentioned fusion protein. As used herein, the terms "fragment", "derivative" and "analog" refer to a polypeptide that substantially retains the function or activity of the fusion protein of the present invention. The polypeptide fragments, derivatives or analogs of the present invention may be (i) a polypeptide in which one or more conservative or non-conservative amino acid residues (preferably conservative amino acid residues) are substituted, or (ii) a polypeptide with a substitution group in one or more amino acid residues, or (iii) a polypeptide formed by fusion of a polypeptide with another compound (such as a compound that prolongs the half-life of polypeptide, such as polyethylene glycol), or (iv) the polypeptide formed by fusion of additional amino acid sequence to this polypeptide sequence (fusion protein formed by fusion with a tag sequence such as leader sequence, secretory sequence or 6His). According to the teachings herein, these fragments, derivatives and analogs fall within the scope well known to those skilled in the art.

A preferred type of active derivative means that compared with the amino acid sequence of the present invention, at most 3, preferably at most 2, and more preferably at most 1 amino acid are replaced by amino acids with close or similar properties to form a polypeptide. These conservative variant polypeptides are best produced according to Table A by performing amino acid substitutions.

**Table A**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides analogs of the fusion protein of the present invention. The difference between these analogs and the polypeptide of the present invention may be a difference in amino acid sequence, may also be a difference in modified form that does not affect the sequence, or both. Analogs also include analogs having residues different from natural L-amino acids (such as D-amino acids), and analogs having non-naturally occurring or synthetic amino acids (such as β, γ-amino acids). It should be understood that the polypeptide of the present invention is not limited to the representative polypeptides exemplified above.

In addition, the fusion protein of the present invention can also be modified. Modification (usually without changing the primary structure) forms include: chemically derivative forms of polypeptides *in vivo* or *in vitro,* such as acetylation or carboxylation. Modifications also include glycosylation, such as those polypeptides produced by glycosylation modifications during the synthesis and processing of the polypeptide or during further processing steps. This modification can be accomplished by exposing the polypeptide to an enzyme that performs glycosylation (such as a mammalian glycosylase or deglycosylase). Modification forms also include sequences with phosphorylated amino acid residues (such as phosphotyrosine, phosphoserine, phosphothreonine). It also includes polypeptides that have been modified to improve their anti-proteolytic properties or optimize their solubility properties.

The term "polynucleotide encoding the fusion protein of the present invention" may include a polynucleotide encoding the fusion protein of the present invention, or a polynucleotide that also includes additional coding and/or non-coding sequences.

The present invention also relates to variants of the above-mentioned polynucleotides, which encode fragments, analogs and derivatives of polypeptides or fusion proteins having the same amino acid sequence as the present invention. These nucleotide variants include substitution variants, deletion variants and insertion variants. As known in the art, an allelic variant is an alternative form of polynucleotide. It may be a substitution, deletion or insertion of one or more nucleotides, but will not substantially change the function of the encoded fusion protein.

The present invention also relates to polynucleotides that hybridize with the aforementioned sequences and have at least 50%, preferably at least 70%, and more preferably at least 80% identity between the two sequences. The present invention particularly relates to polynucleotides that can hybridize with the polynucleotide of the present invention under strict conditions (or stringent conditions). In the present invention, "strict conditions" refer to: (1) hybridization and elution at lower ionic strength and higher temperature, such as 0.2×SSC, 0.1% SDS, 60°C; or (2) adding denaturant during hybridization, such as 50% (v/v) formamide, 0.1% calf serum/0.1% Ficoll, 42°C, etc.; or (3) hybridization occurs only when the identity between the two sequences is at least 90% or more, and more preferably 95% or more.

The fusion protein and polynucleotide of the present invention are preferably provided in an isolated form, and more preferably, are purified to homogeneity.

The full-length sequence of the polynucleotide of the present invention can usually be obtained by PCR amplification method, recombination method or artificial synthesis method. For the PCR amplification method, primers can be designed according to the relevant nucleotide sequence disclosed in the present invention, especially the open reading frame sequence, and a commercially available cDNA library or a cDNA library prepared according to a conventional method known to those skilled in the art is used as a template to amplify the relevant sequence. When the sequence is long, it is often necessary to perform two or more rounds of PCR amplifications, and then each amplified fragments are spliced together in the correct order.

Once the relevant sequences are obtained, the relevant sequences can be obtained in large quantities by recombination method. It is usually cloned into a vector, then transferred into a cell, and then the relevant sequence is isolated from the host cell after proliferation by conventional methods.

In addition, the relevant sequences can also be synthesized by artificial synthesis, especially when the fragment length is short. Usually, by first synthesizing multiple small fragments, and then ligating to obtain very long fragments.

At present, the DNA sequence encoding the protein (or fragment or derivative thereof) of the present invention can be obtained completely through chemical synthesis. The DNA sequence can then be introduced into various existing DNA molecules (or such as vectors) and cells known in the art.

The method of using PCR technology to amplify DNA/RNA is preferably used to obtain the polynucleotide of the present invention. Especially when it is difficult to obtain full-length cDNA from the library, the RACE method (RACE-cDNA end rapid amplification method) can be preferably used, and the primers used for PCR can be appropriately selected according to the sequence information of the present invention disclosed herein, and can be synthesized by conventional methods. The amplified DNA/RNA fragments can be separated and purified by conventional methods such as gel electrophoresis.

### Expression Vector

The present invention also relates to a vector containing the polynucleotide of the present invention, a host cell produced by genetic engineering using the vector of the present invention or the sequence encoding the fusion protein of the present invention, and a method for producing the polypeptide of the present invention through recombinant technology.

Through conventional recombinant DNA technology, the polynucleotide sequence of the present invention can be used to express or produce recombinant fusion protein. Generally, there are the following steps:
(1). using the polynucleotide (or variant) of the present invention encoding the fusion protein of the present invention, or using a recombinant expression vector containing the polynucleotide to transform or transduce a suitable host cell;
(2). culturing the host cell in a suitable medium;
(3). isolating and purifying protein from culture medium or cells.

In the present invention, the polynucleotide sequence encoding the fusion protein can be inserted into a recombinant expression vector. The term "recombinant expression vector" refers to bacterial plasmids, bacteriophages, yeast plasmids, plant cell viruses, mammalian cell viruses such as adenovirus, retrovirus or other vectors well known in the art. Any plasmid and vector can be used as long as it can be replicated and stabilized in the host. An important feature of an expression vector is that it usually contains an origin of replication, a promoter, a marker gene, and translation control elements.

Methods well known to those skilled in the art can be used to construct an expression vector containing the DNA sequence encoding the fusion protein of the present invention and appropriate transcription/translation control signals. These methods include *in vitro* recombinant DNA technology, DNA synthesis technology, and *in vivo* recombination technology. The DNA sequence can be effectively linked to an appropriate promoter in the expression vector to guide mRNA synthesis. Representative examples of these promoters are: *Escherichia coli* lac or trp promoter; lambda phage PL promoter; eukaryotic promoters including CMV immediate early promoter, HSV thymidine kinase promoter, early and late SV40 promoter, retroviral LTRs and some other known promoters that can control gene expression in prokaryotic or eukaryotic cells or viruses. The expression vector also includes a ribosome binding site for translation initiation and a transcription terminator.

In addition, the expression vector preferably contains one or more selectable marker genes to provide phenotypic traits for selecting transformed host cells, such as dihydrofolate reductase, neomycin resistance, and green fluorescent protein (GFP) for eukaryotic cell culture, or tetracycline or ampicillin resistance for *E. coli.*

A vector containing the above-mentioned appropriate DNA sequence and an appropriate promoter or control sequence can be used to transform an appropriate host cell so that it can express the protein.

The host cell can be a prokaryotic cell, such as a bacterial cell; or a lower eukaryotic cell, such as a yeast cell; or a higher eukaryotic cell, such as a mammalian cell. Representative examples include: *Escherichia coli, Streptomyces;* bacterial cells of *Salmonella typhimurium;* fungal cells such as yeast and plant cells (such as ginseng cells).

When the polynucleotide of the present invention is expressed in higher eukaryotic cells, if an enhancer sequence is inserted into the vector, the transcription will be enhanced. Enhancers are cis-acting factors of DNA, usually about 10 to 300 base pairs, acting on promoters to enhance gene transcription. Examples include the 100 to 270 base pair SV40 enhancer on the late side of the replication initiation point, the polyoma enhancer on the late side of the replication initiation point, and adenovirus enhancers and the like.

Those of ordinary skill in the art know how to select appropriate vectors, promoters, enhancers and host cells.

Transformation of host cells with recombinant DNA can be carried out by conventional techniques well known to those skilled in the art. When the host is a prokaryote such as *Escherichia coli,* competent cells that can absorb DNA can be harvested after the exponential growth phase and treated with the CaCl₂ method. The steps used are well known in the art. Another method is to use MgCl₂. If necessary, transformation can also be carried out by electroporation. When the host is a eukaryote, the following DNA transfection methods can be selected: calcium phosphate co-precipitation method, conventional mechanical methods such as microinjection, electroporation, liposome packaging, etc..

The obtained transformants can be cultured by conventional methods to express the polypeptide encoded by the gene of the present invention. Depending on the host cell used, the medium used in the culture can be selected from various conventional mediums. The culture is carried out under conditions suitable for the growth of the host cell. After the host cells have grown to an appropriate cell density, the selected promoter is induced by a suitable method (such as temperature conversion or chemical induction), and the cells are then cultured for a period of time.

The recombinant polypeptide in the above method can be expressed in the cell, on the cell membrane, or secreted out of the cell. If necessary, the physical, chemical, and other characteristics can be used to separate and purify the recombinant protein through various separation methods. These methods are well known to those skilled in the art. Examples of these methods include, but are not limited to: conventional renaturation treatment, treatment with protein precipitation agent (salting out method), centrifugation, bacteria broken through osmosis, ultra treatment, ultracentrifugation, molecular sieve chromatography (gel filtration), adsorption chromatography, ion exchange chromatography, high performance liquid chromatography (HPLC) and various other liquid chromatography techniques and combinations of these methods.

### Construction of the semaglutide expression vector

The FP-TEV-EK-GLP1 (with Boc modification at position 18, 17, 16, 15 or 14) fragment which contains the target gene was synthesized, of which the two ends had the recognition sites of restriction endonucleases Nco I and Xho I. The codon of this sequence was optimized and can achieve high level expression of functional protein in *E. coli.* After expression, the restriction endonucleases Nco I and Xho I were used to cut the expression vector "pBAD/His A(Kana^{R})" and the plasmid containing the target gene "FP-TEV-EK-GLP1 (18, 17, 16, 15 or 14)". The digested products were separated by agarose electrophoresis, and then extracted by agarose gel DNA recovery kit. Finally, the two DNA fragments were connected by T4 DNA ligase. The connected product was chemically transformed into *E. coli* Top10 cells, and the transformed cells were cultured in LB agar medium (10 g/L yeast peptone, 5 g/L yeast extract, 10 g/L NaCl, 1.5% agar) containing 50 µ G/mL kanamycin overnight. Three live colonies were picked and cultured in SmL liquid LB medium (10g/L yeast peptone, 5g/L yeast extract and 10g/L NaCl) containing 50µg/mL kanamycin overnight, and the plasmid was extracted by using small amount plasmid extraction kit. Then, the extracted plasmid was sequenced using the sequencing oligonucleotide primer 5'-ATGCCATAGCATTTTTATCC-3' (SEQ ID NO: 15) to confirm correct insertion. The finally obtained plasmid was named as "pBAD-FP-TEV-EK-GLP1 (18, 17, 16, 15 or 14)".

### Fmoc modification

The use of peptides is increasing in the field of biomedicine. Amino acids are the basic raw materials for the peptide synthesis technology. All amino acids contain α-amino and carboxyl groups, and some also contain side chain active groups such as: hydroxyl, amino, guanidyl and heterocyclic. Therefore, it is necessary to protect amino groups and side chain active groups in the peptide-connecting reaction, and remove the protective groups after synthesis of polypeptides, otherwise amino acid misconnection and many side reactions will occur.

Fluorenylmethoxycarbonyl (Fmoc) is a base-sensitive protective group that can be removed in concentrated ammonia or dioxane-methanol-4N NaOH (30:9:1) and 50% dichloromethane solutions of piperidine, ethanolamine, cyclohexylamine, 1,4-dioxane, pyrrolidone and other ammonias.

Under weakly alkaline conditions such as sodium carbonate or sodium bicarbonate, Fmoc-Cl or Fmoc-OSu are generally used to introduce Fmoc protective groups. Compared to Fmoc-Cl, Fmoc-OSu is easier to control reaction conditions and has fewer side reactions.

Fmoc has strong ultraviolet absorption, the maximum absorption wavelength is 267nm (ε18950), 290nm (ε5280), 301nm (ε6200). Thus it can be detected through ultraviolet absorption, which brings many conveniences to the automatic peptide synthesis by instruments. In addition, it can be compatible with a wide range of solvents and reagents, has high mechanical stability, and can be used with a variety of carriers and a variety of activation methods. Therefore, the Fmoc protection groups are most commonly used in peptide synthesis now.

### Fmoc-OSu (9-Fluorenyl methyl succinyl iminocarbonate)

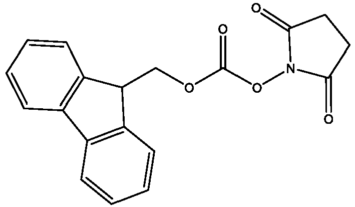

### Side chain of semaglutide

tBuO-Ste-Glu(AEEA-AEEA-OSu)-OtBu is the side chain of semaglutide.

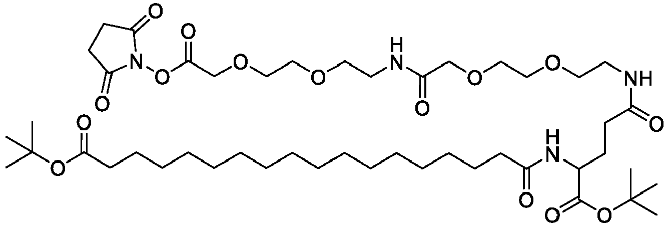

The preparation of semaglutide is to first use genetic recombination technique to obtain the semaglutide main chain with a Boc-protected lysine at position 14, 15, 16, 17 or 18, and then conjugate the semaglutide side chain tBuO-Ste-Glu(AEEA-AEEA-OSu)-OtBu to obtain semaglutide.

### Preparation of semaglutide

The 5 synthesis routes of semaglutide provided by the present invention are set forth in the following Formula A, Formula B, Formula C, Formula D and Formula E, respectively. Fmoc complex-modified Compound 2 is produced from the Boc-semaglutide precursor (Compound 1, 7, 8, 9, 10). Boc protection is removed from Compound 2 to obtain Compound 3. Compound 3 is reacted with activated semaglutide side chain tBuO-Ste-Glu(AEEA-AEEA-OSu)-OtBu to obtain Compound 4. Then Compound 5 is obtained through Fmoc-removing reaction. OtBu protective group is removed from the side chain to finally obtain semaglutide Compound 6.

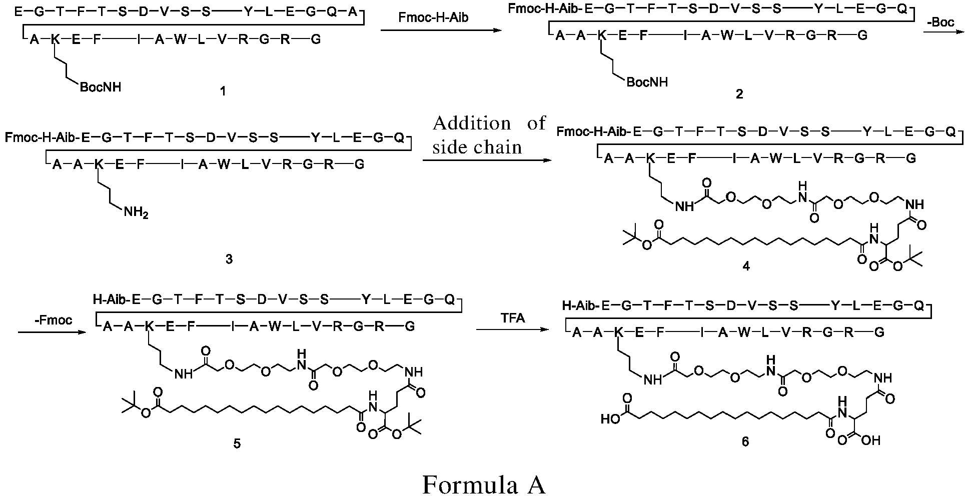

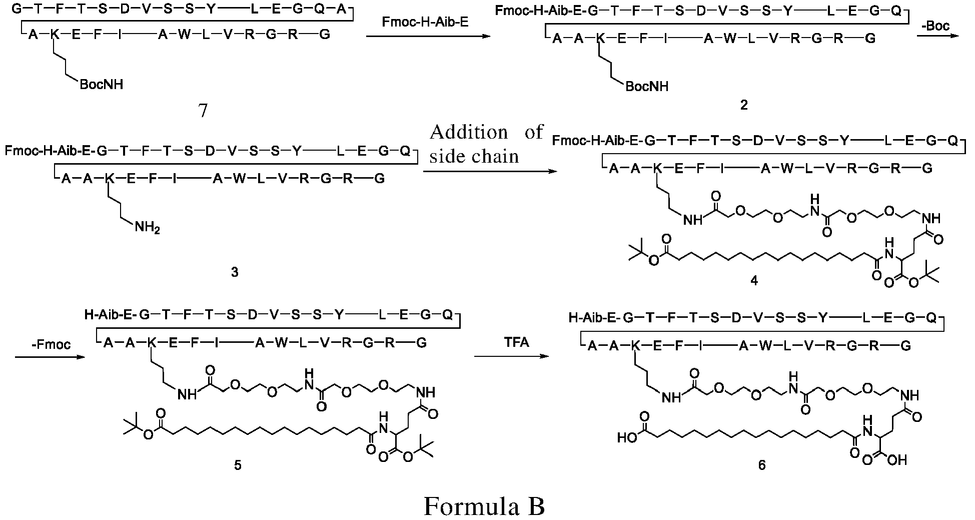

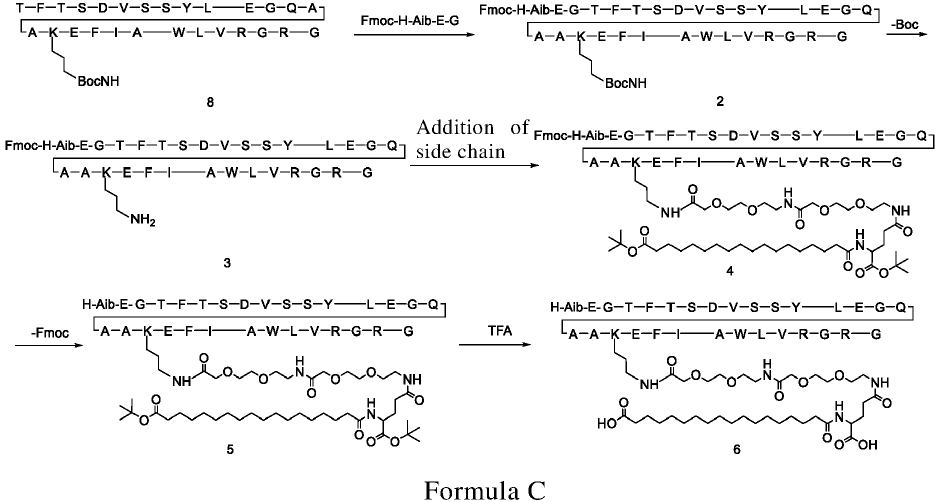

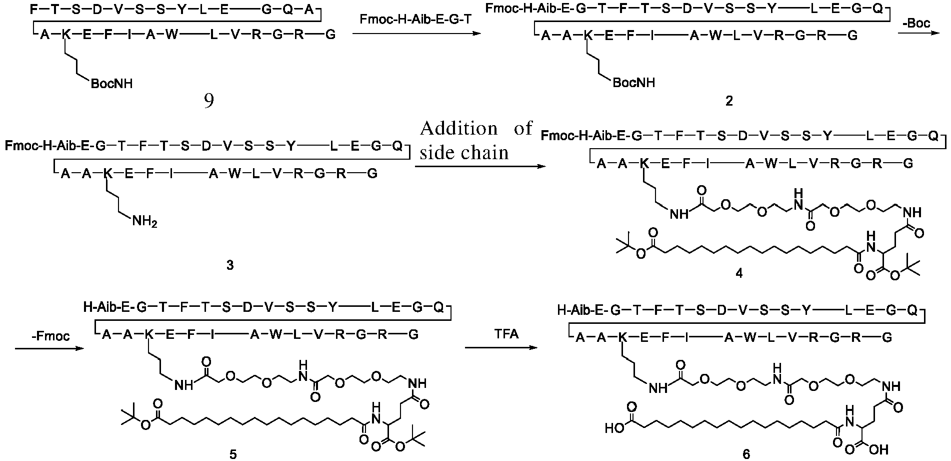

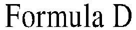

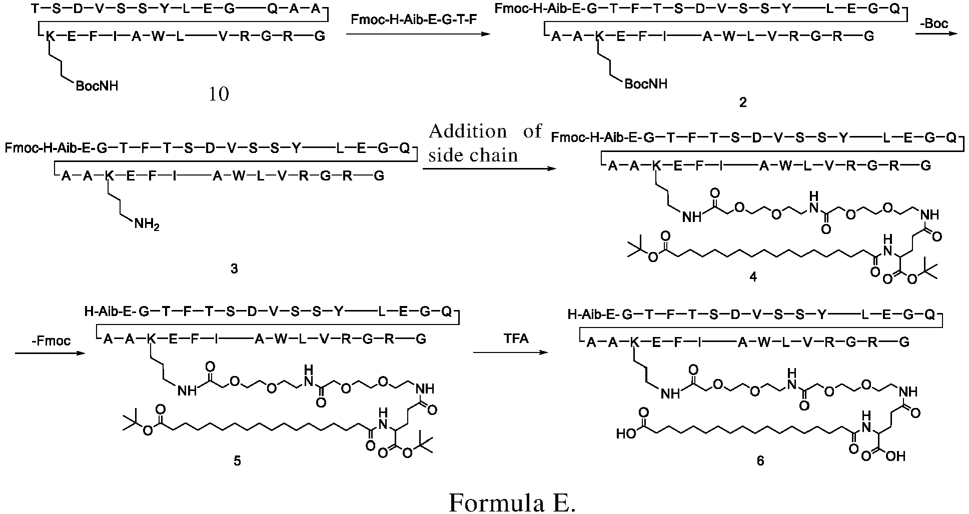

Specifically, the present invention provides a method for preparing semaglutide, comprising the steps:
(i) providing a Boc-modified semaglutide precursor;
(ii) modifying the Boc-modified semaglutide precursor with an activated Fmoc complex, thereby obtaining a Fmoc and Boc-modified semaglutide main chain;
(iii) removing the Boc from the Fmoc and Boc-modified semaglutide main chain, and reacting the same with the semaglutide side chain, thereby obtaining a Fmoc-modified semaglutide; and
(iv) removing the Fmoc from the Fmoc-modified semaglutide and the OtBu from the side chain thereof, thereby obtaining the semaglutide.

### The present invention mainly has the following advantages:

(1) The present invention produces the Boc-modified semaglutide precursor without adopting methods such as dilution, ultrafiltration and liquid replacement to remove excess inorganic salts in the supernatant of the fermentation broth. In the method of the present invention, the one-step yield of isolating Boc-semaglutide precursor by using chromatography column is more than 70%, which is 3 times higher than the conventional method, and the yield of Boc-semaglutide precursor is about 800-1000mg/L. Moreover, the method of the present invention can remove most of the pigment, reduce the conventional multi-step process, process time and equipment investment cost;
(2) Due to the Boc-lysine protection at position 20, the present invention can directly synthesize semaglutide by orthogonal reaction with Fmoc protection.
(3) The semaglutide synthesized through the method of the present invention has no N-terminal fatty acid acylation impurities, which is conducive to downstream purification and reduces costs.
(4) Compared with solid-phase synthesis, the method of the present invention does not produce racemic impurity polypeptides, and does not need to use a large number of modified amino acids, does not use a large number of organic reagents and has little environmental pollution and lower cost;
(5) The fusion protein of the present invention contains a high proportion of semaglutide (the fusion ratio is increased). The FP or A-FP in the fusion protein contains arginine and lysine and can be digested with proteases into small fragments whose molecular weight are quite different from the target protein, and can readily be separated.

The present invention will be further illustrated with reference to the specific examples. It should be understood that these examples are only used to illustrate the present invention and not to limit the scope of the present invention. The experimental methods without specific conditions in the following examples are usually based on conventional conditions, or according to the conditions suggested by the manufacturer. Unless otherwise specified, all percentages and parts are calculated by weight.

### Example 1 Construction of the semaglutide expression bacterial strain

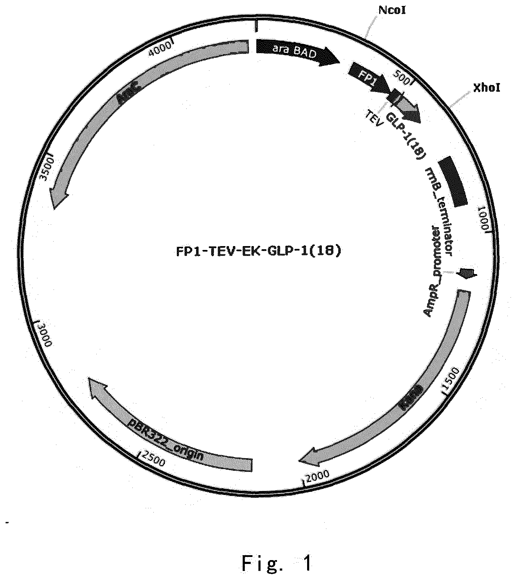

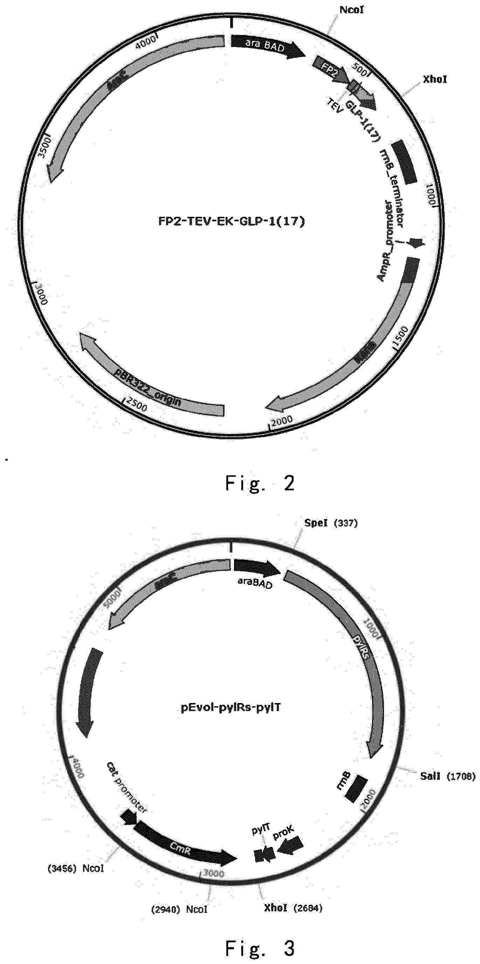

The construction of the semaglutide expression plasmid refers to the description of Examples in Chinese patent application No. 201910210102.9. The DNA fragments of the fusion proteins FP1-TEV-EK-GLP-1(18, 17, 16, 15, 14) were cloned to the NcoI-XhoI site downstream of the araBAD promoter of the expression vector plasmid pBAD/His A (purchased from NTCC, kanamycin resistance) to obtain the plasmid pBAD-FP1-TEV-EK-GLP-1(18) or pBAD-FP2-TEV-EK-GLP-1(17), pBAD-FP2-TEV-EK-GLP-1(16), pBAD-FP2-TEV-EK-GLP-1(15), pBAD-FP2-TEV-EK-GLP-1(14). Among them, the plasmid maps of pBAD-FP1-TEV-EK-GLP-1(18) or pBAD-FP2-TEV-EK-GLP-1(17) are shown in Figures 1 and 2.

Based on the semaglutide precursors with 2-7 amino acids deleted at the N-terminus respectively as shown in SEQ ID NOs: 1, 2, 23, 24 and 25, Fusion protein 1, Fusion protein 2, Fusion protein 3, Fusion protein 4 and Fusion protein 5 were constructed.

The amino acid sequence of the Fusion Protein 1 is as shown in SEQ ID NO. 4:

MVSKGEELFTGVKLTLKFICTTYVQERTISFKDTYKTRAEVKFEGD
ENLYFQGDDDDKEGTFTSDVSSYLEGQAAKEFIAWLVRGRG

The amino acid sequence of the Fusion Protein 2 is as shown in SEQ ID NO: 5:

MVSKGEELFTGVYVQERTISFKDTYKTRAEVKFEGDTLVNRIELKGIDF
ENLYFQGDDDDKGTFTSDVSSYLEGQAAKEFIAWLVRGRG

The amino acid sequence of the Fusion Protein 3 is as shown in SEQ ID NO: 26:

MVSKGEELFTGVYVQERTISFKDTYKTRAEVKFEGDTLVNRIELKGIDF
ENLYFQGDDDDKTFTSDVSSYLEGQAAKEFIAWLVRGRG

The amino acid sequence of the Fusion Protein 4 is as shown in SEQ ID NO: 27:

MVSKGEELFTGVYVQERTISFKDTYKTRAEVKFEGDTLVNRIELKGIDF
ENLYFQGDDDDKFTSDVSSYLEGQAAKEFIAWLVRGRG

The amino acid sequence of the Fusion Protein 5 is as shown in SEQ ID NO: 28:

MVSKGEELFTGVKLTLKFICTTYVQERTISFKDTYKTRAEVKFEGD
ENLYFQGDDDDKTSDVSSYLEGQAAKEFIAWLVRGRG.

Among them the sequence of the leader peptide is MVSKGEELFTGV (SEQ ID NO: 7).

The sequence of the green fluorescent protein folding unit (FP) is
FP1: KLTLKFICTTYVQERTISFKDTYKTRAEVKFEGD (SEQ ID NO: 6, U3-U4-U5)
FP2: YVQERTISFKDTYKTRAEVKFEGDTLVNRIELKGIDF (SEQ ID NO: 10, U4-U5-U6)
the restriction site of TEV enzyme is ENLYFQG (SEQ ID NO: 8);
the restriction site of enterokinase is DDDDK (SEQ ID NO: 9);

The amino acid sequences of the semaglutide precursor with 2-7 amino acids deleted at the N-terminus are shown in SEQ ID NOs: 1, 2, 23, 24, and 25, respectively.
SEQ ID NO: 1: EGTFTSDVSSYLEGQAA**K**EFIAWLVRGRG
SEQ ID NO: 2: GTFTSDVSSYLEGQAA**K**EFIAWLVRGRG
SEQ ID NO: 23: TFTSDVSSYLEGQAA**K**EFIAWLVRGRG
SEQ ID NO: 24: FTSDVSSYLEGQAA**K**EFIAWLVRGRG
SEQ ID NO: 25: GTFTSDVSSYLEGQAA**K**EFIAWLVRGRG
(K is the Boc-modified lysine).

Then the DNA sequence of pylRs was cloned to the SpeI-SalI site downstream of the araBAR promoter of the expression vector plasmid pEvol-pBpF (purchased from NTCC, chloramphenicol resistance), and the DNA sequence of the tRNA (pylTcua) of lysyl-tRNA synthase was inserted downstream of the proK promoter by PCR. The plasmid is named as pEvol-pylRs-pylT. The plasmid map is shown in Figure 3.

The constructed plasmid pBAD-FP1-TEV-EK-GLP-1(18) and pEvol-pylRs-pylT were co-transformed into *E. coli* TOP10 strains. The recombinant strains that express the semaglutide fusion protein FP-TEV-EK-GLP-1(18) were screened and obtained.

The constructed plasmid pBAD-FP1-TEV-EK-GLP-1(17) and pEvol-pylRs-pylT were co-transformed into *E. coli* TOP10 strain. The recombinant strain expressing the semaglutide fusion protein FP-TEV-EK-GLP-1(17) was screened and obtained.

The constructed plasmid pBAD-FP1-TEV-EK-GLP-1(16) and pEvol-pylRs-pylT were co-transformed into *E. coli* TOP10 strain. The recombinant strain expressing the semaglutide fusion protein FP-TEV-EK-GLP-1(16) was screened and obtained.

### Example 2 Expression of Boc-semaglutide precursor

Three kinds of recombinant *E. coli* seed liquid were inoculated into fermentation medium (yeast peptone, yeast extract powder, glycerol, Boc-L-lysine, buffer and micronutrients) at an amount of 5% (V/V) respectively, cultured in batches at 37°C, pH 7.0, until pH reached to 7.05. Carbon and nitrogen materials were fed separately, and carbon and nitrogen materials were fluidly added according to the constant pH method. After feeding, 7.5 M ammonia water was automatically fluidly added, and the pH was controlled at 7.0-7.2. After incubation for 4-6 hours, 2.5g/L of L-arabinose was added for induction for 14±2 hours. Three fermentation broths containing semaglutide precursor fusion protein were obtained.

### Example 3 Preparation of Boc-semaglutide precursor inclusion bodies

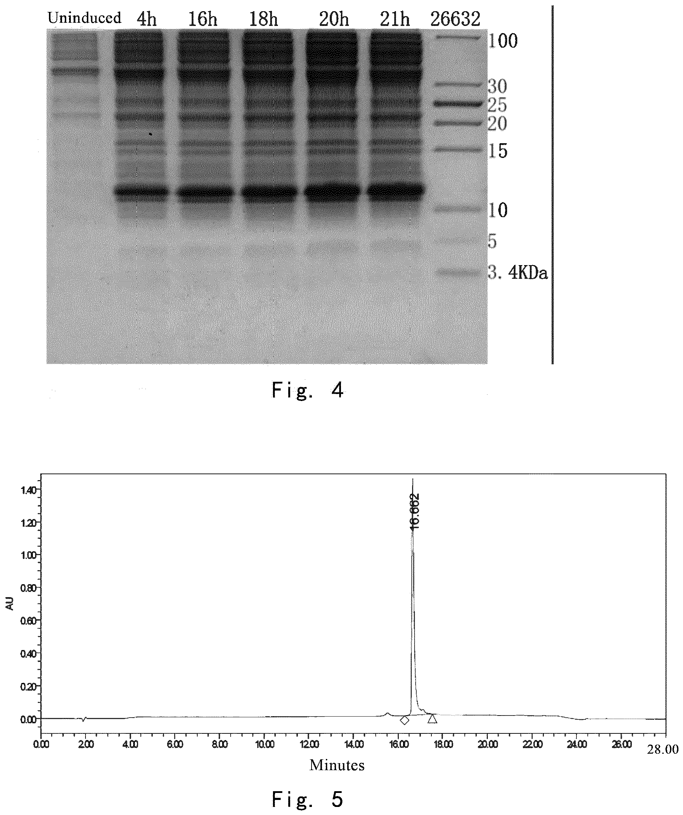

After centrifuging the three fermentation broths obtained in Example 2, the wet bacteria were mixed with the bacteria-breaking buffer (0.5-1.5% (ml/ml) Tween 80, 1mmol/L EDTA-2Na and 100mmol/L NaCl) in a volume ratio of 1:1, suspended for 3 h, and then broken by a high-pressure homogenizer (800 ± 50 bar, 6~20 °C). The inclusion bodies were collected by centrifugation after the bacteria were broken. The inclusion bodies were washed with buffer and then weighed. The yields of inclusion bodies of Fusion proteins 1, 2, 3 were 39-43g/L, 41-45g/L and 40-43g/L, respectively. The result of SDS-PAGE electrophoresis for Fusion protein 1 is shown in Figure 4.

### Example 4 Denaturation and Renaturation and digestion of Boc-semaglutide precursor inclusion bodies

8mol/L urea dissolved buffer was added into the inclusion bodies obtained in Example 3 at a mass-volume ratio of 1:15, stirred and dissolved at room temperature. The concentration of protein was determined via Bradford method. The total protein concentration of the inclusion body dissolved solution was controlled at 20 mg/mL, pH of that was adjusted to 9.0±1.0 using NaOH. The inclusion body dissolved solution was dripped into the renaturation buffer containing 5-20mmol/L sodium carbonate, 5-20mmol/L glycine, 0.3-0.5 mmol/L EDTA-2Na to dilute the inclusion body dissolved solution to 5-10 times and renature the same. The pH value of the fusion protein renaturation solution was maintained at 9.0-10.0, and the temperature was controlled at 4-8 °C. The renaturation time was 10-20h.

The results show that after dissolution, the proportion of Fusion protein 1 and Fusion protein 2 is about 30% and 33%, and the proportion of Fusion protein 3 is about 31%.

### Example 5 Preliminary purification of the Boc-semaglutide precursor fusion protein

The fusion protein renaturation solution obtained in Example 4 was filtered through a 0.45 µm filter membrane to remove the undissolved substance. According to the difference of protein isoelectric points, the Q anion exchange column was used to preliminarily purify the fusion protein.

The experimental results show that, after anion exchange chromatography, the purity of Boc-semaglutide precursor fusion proteins 1, 2 and 3 all reach over 65%, the loading capacity is about 18mg/mL, and the yield is greater than 80%.

### Example 6 Digestion of the Boc-semaglutide precursor fusion protein

The Boc-semaglutide precursor fusion protein preliminarily purified in Example 5 was desalted and adjusted to the pH of 7.5-8.5. The temperature was controlled at 18-25 °C, and 0.3-0.5U/mg enterokinase was added for digestion for 8-24h to obtain the Boc-semaglutide precursor. The Boc-semaglutide precursor 1, precursor 2 and precursor 3 were about 0.9g/L and 1.2g/L, 1.0g/L, and the digestion efficiency was ≥95%.

### Example 7 Reverse phase chromatography of Boc-semaglutide precursor

According to the hydrophobicity difference of peptides and proteins, the Boc-semaglutide precursor was purified by C8 reverse phase chromatography to remove most of heteroproteins.

The digestion solution of Boc-semaglutide precursor 1, precursor 2 and precursor 3 obtained in Example 6 was added with 3M hydrochloric acid to adjust the pH value of the sample to 2.0-3.0. The sample was added with acetonitrile so that the concentration of acetonitrile in the sample was 10% (v/v), filtrated with 0.45µm filter membrane and reserved, and then performed with reverse phase chromatography for separation and purification.

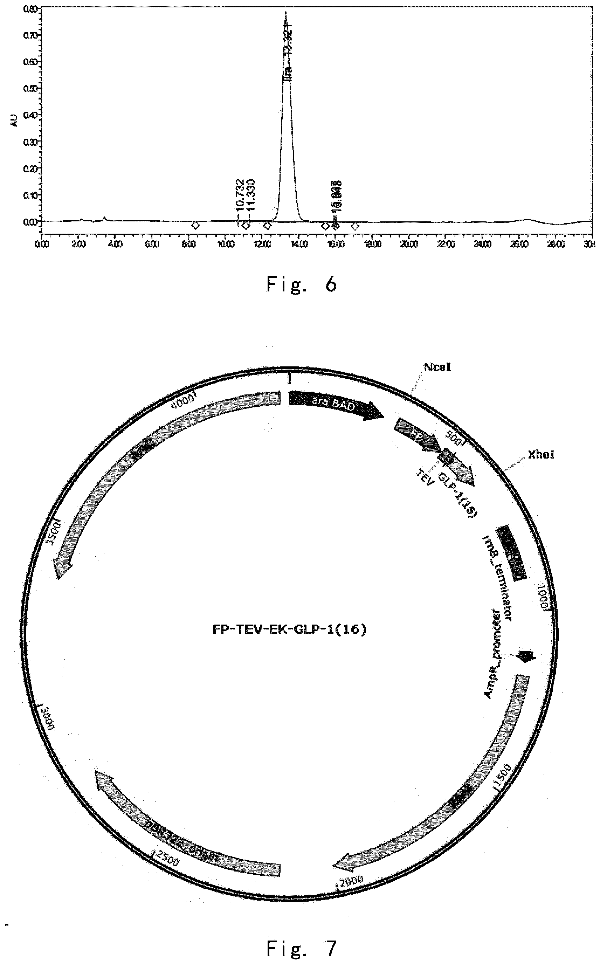

The aqueous solution containing trifluoroacetic acid was used as mobile phase A; and acetonitrile solution containing trifluoroacetic acid was used as mobile phase B. Boc-semaglutide precursor was combined with the filler and the loading amount of Boc-semaglutide precursor was controlled no higher than 10 mg/mL. Gradient elution was conducted to collect Boc-semaglutide precursor. The experimental results show that the purity of Boc-semaglutide precursor 1, precursor 2 and precursor 3 collected through reverse-phase chromatography is ≥ 90%, and the yield is greater than 80%. The HPLC detection spectrogram of Boc-semaglutide precursor 1 after purification is shown in Figure 5, and the HPLC detection spectrogram of precursor 3 is shown in Figure 6. The molecular weights of Boc-semaglutide precursors 1, 2 and 3 determined by mass spectrometry are consistent with the theoretical values, respectively.

### Example 8 Preparation of semaglutide using Boc-semaglutide precursor 1 (Fmoc-H-Aib, Route 1)

The Boc-semaglutide precursor 1 (Compound 1, the molar ratio of materials take 30mg for example) obtained in Example 7 was taken and added with activated Fmoc-H-Aib, DIPEA and DMF according to the molar ratio in Table 1, and reacted for 8-12 hours to prepare the Fmoc and Boc-protected semaglutide main chain. Among them, the Fmoc-H-Aib is an Fmoc-H-Aib in the form of an activated ester, formed by HOSu/DCC activation, in which the Aib amino acid is attached with an OSu group. Then mixed solution of methyl tert-butyl ether/petroleum ether (3:1) at 0 ± 5 °C was added to the reaction solution, precipitated and centrifuged. The precipitation was washed with methyl tert-butyl ether for 2-3 times to obtain the Fmoc-protected Compound 2: Fmoc- GLP-1(Lys²⁰Boc).

**Table 1 Molar ratio of materials**

| | Boc-semaglutide | Fmoc-H-Aib | DIPEA | DMF |
|---|---|---|---|---|
| Equivalent or volume | 1.0 eq | 2.5eq | 12eq | 1V |

Compound 2 was added to the precooled TFA solution at 0 ± 5 °C, stirred for 0.5-2.0h. 15-20 times the volume of mixed solution of methyl tert-butyl ether and petroleum ether (3:1) at 0 ± 5°C was added to the reaction system, precipitated and centrifuged. The precipitation was washed with the mixed solution 2~3 times to finally obtain the Boc-removed solid Compound 3: Fmoc- GLP-1(Lys²⁰NH₂).

The Boc-removed Compound 3 was added with DMF and 12eq DIPEA, and stirred gently at room temperature for 5 min. 2.5eq of tBuO-Ste-Glu(AEEA-AEEA-OSu)-OtBu was dissolved in DMF solution and added to the obtained mixture, and the reaction mixture was gently shaken for 2-3 h at room temperature. The mixed solution of methyl tert-butyl ether and petroleum ether (3:1) at 0 ± 5 °C was added to the reaction system at 15-20 times of the volume of the reaction system, precipitated and centrifuged. The solid was washed 2-3 times with the mixed solution, dried in vacuum to obtain Compound 4: Fmoc-GLP-1-(tBuO-Ste-Glu(AEEA-AEEA)-OtBu)(20).

Compound 4 was taken and added into DMF solution containing 20% piperidine, and reacted at room temperature for 0.5-2.0 hours. Then mixed solvent of methyl tert-butyl ether and petroleum ether (3:1) at 0 ± 5 °C was added to the reaction system, precipitated and centrifuged. The solid was washed with mixed solvent of methyl tert-butyl ether and petroleum ether (3:1) for 3-5 times to obtain the Fmoc-removed Compound 5: GLP-1-(tBuO-Ste-Glu(AEEA-AEEA)-OtBu)(20).

Compound 5 was taken and added into a mixed solution of TFA (trifluoroacetic acid), TIS (triisopropylsilane) and DCM (dichloromethane) ((90% TFA: 10% TIS): DCM=1:2), and shaking reacted at room temperature for 2-4 hours to remove the OtBu protective group on side chain. 10-20 times volume of mixed solvent of methyl tert-butyl ether and petroleum ether (3:1) at 0 ± 5 °C was added to the reaction system, precipitated and centrifuged. The solid was washed with the mixed solvent of methyl tert-butyl ether and petroleum ether (3:1) for 3 times to obtain the final product. After HPLC purification, the semaglutide with purity over 98% was obtained.

### Example 9 Preparation of semaglutide using Boc-semaglutide precursor 2 (Fmoc-H-Aib-E, Route 2)

The Boc-semaglutide precursor 2 (Compound 7, the molar ratio of materials take 30mg for example) obtained in Example 7 was taken and added with activated Fmoc-H-Aib-E, DIPEA and DMF according to the molar ratio in Table 2, and reacted for 8-12 hours to prepare the Fmoc and Boc-protected semaglutide main chain. Among them, the Fmoc-H-Aib-E is in the form of an activated ester, formed by HOSu/DCC activation. The mixed solution of methyl tert-butyl ether/petroleum ether (3:1) at 0 ± 5 °C was added to the reaction solution, precipitated and centrifuged. The precipitation was washed 2-3 times to obtain the Fmoc-protected Compound 2: Fmoc- GLP-1(Lys²⁰Boc).

**Table 2 Molar ratio of materials**

| | Boc-semaglutide | Fmoc-H-Aib-E | DIPEA | DMF |
|---|---|---|---|---|
| Equivalent or volume | 1.0 eq | 2.5eq | 12eq | 1V |

Compound 2 was added to the precooled TFA solution at 0 ± 5 °C, stirred for 0.5-2.0h. The mixed solution of methyl tert-butyl ether and petroleum ether (3:1) at 0±5°C was added to the reaction system at 15-20 times of the volume of the reaction system, precipitated and centrifuged. The precipitation was washed with the mixed solution 2~3 times to finally obtain the Boc-removed solid Compound 3: Fmoc- GLP-1(Lys²⁰NH₂).

The Boc-removed Compound 3 was added with DMF and 12eq DIPEA, and stirred gently at room temperature for 5 min. 2.5eq of tBuO-Ste-Glu(AEEA-AEEA-OSu)-OtBu was dissolved in DMF solution and added to the obtained mixture, and the reaction mixture was gently shaken for 2-3 h at room temperature. The mixed solution of methyl tert-butyl ether and petroleum ether (3:1) at 0 ± 5 °C was added to the reaction system at 15-20 times of the volume of the reaction system, precipitated and centrifuged. The solid was washed 2-3 times with the mixed solution, dried in vacuum to obtain Compound 4: Fmoc-GLP-1-(tBuO-Ste-Glu(AEEA-AEEA)-OtB u) (20).

Compound 4 was taken and added into DMF solution containing 20% piperidine, and reacted at room temperature for 0.5-2.0 hours. Then mixed solvent of methyl tert-butyl ether and petroleum ether (3:1) at 0 ± 5 °C was added to the reaction system, precipitated and centrifuged. The solid was washed with mixed solvent of methyl tert-butyl ether and petroleum ether (3:1) for 3-5 times to obtain the Fmoc-removed Compound 5: GLP-1-(tBuO-Ste-Glu(AEEA-AEEA)-OtBu)(20).

Compound 5 was taken and added into a mixed solution of TFA (trifluoroacetic acid), TIS (triisopropylsilane) and DCM (dichloromethane) ((90% TFA: 10% TIS): DCM=1:2), and shaking reacted at room temperature for 2-4 hours to remove the OtBu protective group on side chain. 10-20 times volume of mixed solvent of methyl tert-butyl ether and petroleum ether (3:1) at 0 ± 5 °C was added to the reaction system, precipitated and centrifuged. The solid was washed with the mixed solvent of methyl tert-butyl ether and petroleum ether (3:1) for 3 times to obtain the final product. After HPLC purification, the semaglutide with purity over 98% was obtained.

### Example 10 Preparation of semaglutide using Boc-semaglutide precursor 2 (Fmoc-H-Aib-E-G, Route 3)

The Boc-semaglutide precursor 3 (Compound 8, the molar ratio of materials take 30mg for example) obtained in Example 7 was taken and added with activated Fmoc-H-Aib-E-G, DIPEA and DMF according to the molar ratio in Table 3, and reacted for 8-12 hours to prepare the Fmoc and Boc-protected semaglutide main chain. Among them, the Fmoc-H-Aib-E-G is in the form of an activated ester, formed by HOSu/DCC activation. Then mixed solution of methyl tert-butyl ether/petroleum ether (3:1) at 0 ± 5 °C was added to the reaction solution, precipitated and centrifuged. The precipitation was washed with methyl tert-butyl ether for 2-3 times for crude purification, to obtain the Fmoc and Boc-protected Compound 2: moc- GLP-1(Lys²⁰Boc).

**Table 3 Molar ratio of materials**

| | Boc-semaglutide precursor | Fmoc-H-Aib-E-G | DIPEA | DMF |
|---|---|---|---|---|
| Equivalent or volume | 1.0 eq | 2.5eq | 12eq | 1V |

Compound 2 was added to the precooled TFA solution at 0 ± 5 °C, stirred for 0.5-2.0h. The mixed solution of methyl tert-butyl ether and petroleum ether (3:1) at 0 ± 5°C was added to the reaction system at 15-20 times of the volume of the reaction system, precipitated and centrifuged. The precipitation was washed with the mixed solution 2~3 times to finally obtain the Boc-removed solid Compound 3: Fmoc- GLP-1(Lys²⁰NH₂).

The Boc-removed Compound 3 was added with DMF and 12eq DIPEA, and stirred gently at room temperature for 5 min. 2.5eq of tBuO-Ste-Glu(AEEA-AEEA-OSu)-OtBu was dissolved in DMF solution and added to the obtained mixture, and the reaction mixture was gently shaken for 2-3 h at room temperature. The mixed solution of methyl tert-butyl ether and petroleum ether (3:1) at 0 ± 5 °C was added to the reaction system at 15-20 times of the volume of the reaction system, precipitated and centrifuged. The solid was washed 2-3 times with the mixed solution, dried in vacuum to obtain Compound 4: Fmoc-GLP-1-(tBuO-Ste-Glu(AEEA-AEEA)-OtBu)(20).

Compound 4 was taken and added into DMF solution containing 20% piperidine, and reacted at room temperature for 0.5-2.0 hours. Then mixed solvent of methyl tert-butyl ether and petroleum ether (3:1) at 0 ± 5 °C was added to the reaction system, precipitated and centrifuged. The solid was washed with mixed solvent of methyl tert-butyl ether and petroleum ether (3:1) for 3-5 times to obtain the Fmoc-removed Compound 5: GLP-1-(tBuO-Ste-Glu(AEEA-AEEA)-OtBu)(20).

Compound 5 was taken and added into a mixed solution of TFA (trifluoroacetic acid), TIS (triisopropylsilane) and DCM (dichloromethane) ((90% TFA: 10% TIS): DCM=1:2), and shaking reacted at room temperature for 2-4 hours to remove the OtBu protective group on side chain. 10-20 times volume of mixed solvent of methyl tert-butyl ether and petroleum ether (3:1) at 0 ± 5 °C was added to the reaction system, precipitated and centrifuged. The solid was washed with the mixed solvent of methyl tert-butyl ether and petroleum ether (3:1) for 3 times to obtain the final product. After HPLC purification, the semaglutide with purity over 98% was obtained.

### Comparative Example

The construction and expression of the fusion protein expression strain was carried out by using a method similar to that in Example 1-3, wherein the difference was merely that the amino acid sequence of the fusion protein used for expression is as shown in SEQ ID NO: 22.

MKKLLFAIPLVVPFYSHSTMELEICSWYHMGIRSFLEQKLISEEDLNSAV
DDDDDKEGTFTSDVSSYLEGQAAKEFIAWLVRGRG (SEQ ID NO: 22)

The above fusion protein contains a gIII signal peptide. The results show that the yield of inclusion bodies was 30g wet weight inclusion bodies. The above results show that, compared with the expression of conventional structural fusion protein, the expression amount of the fusion protein of the present invention is significantly increased.

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents are equally within the scope of the claims appended to this application.

## Claims

1. A semaglutide precursor fusion protein having the structure as shown in Formula I from N-terminus to the C-terminus:
A-FP-TEV-EK-G (I)
wherein,
"-" represents a peptide bond;
A is absent or a leader peptide sequence,
FP is a green fluorescent protein folding unit;
TEV is the first restriction site, and preferably is a restriction site of TEV enzyme (as shown in sequence ENLYFQG, SEQ ID NO: 8);
EK is the second restriction site, and preferably is a restriction site of enterokinase (as shown in sequence DDDDK, SEQ ID NO: 9);
G is a semaglutide precursor or a fragment thereof;
wherein the green fluorescent protein folding unit comprises 2-6 β-folding units selected from the group consisting of:
| β-folding unit | | Amino acid sequence |
|---|---|---|
| | u1 | VPILVELDGDVNG (SEQ ID NO: 11) |
| | u2 | HKFSVRGEGEGDAT (SEQ ID NO: 12) |
| | u3 | KLTLKFICTT (SEQ ID NO: 13) |
| | u4 | YVQERTISFKD (SEQ ID NO: 14) |
| | u5 | TYKTRAEVKFEGD (SEQ ID NO: 15) |
| | u6 | TLVNRIELKGIDF (SEQ ID NO: 16) |
| | u7 | HNVYITADKQ (SEQ ID NO: 17) |
| | u8 | GIKANFKIRHNVED (SEQ ID NO: 18) |
| | u9 | VQLADHYQQNTPIG (SEQ ID NO: 19) |
| | u10 | HYLSTQSVLSKD (SEQ ID NO: 20) |
| | u11 | HMVLLEFVTAAGI (SEQ ID NO: 21) . |

2. The fusion protein according to claim 1, wherein the green fluorescent protein folding unit is u2-u3, u4-u5, u1-u2-u3, u3-u4-u5 or u4-u5-u6.

3. The fusion protein according to claim 1, wherein the G is a Boc-modified semaglutide precursor comprising:
a first semaglutide precursor modified with Boc at position 18, whose amino acid sequence is as shown in SEQ ID NO: 1;
or, a second semaglutide precursor modified with Boc at position 17, whose amino acid sequence is as shown in SEQ ID NO: 2;
or, a third semaglutide precursor modified with Boc at position 16, whose amino acid sequence is as shown in SEQ ID NO: 23;
or, a fourth semaglutide precursor modified with Boc at position 15, whose amino acid sequence is as shown in SEQ ID NO: 24;
or, a fifth semaglutide precursor modified with Boc at position 14, whose amino acid sequence is as shown in SEQ ID NO: 25.

4. The fusion protein according to claim 1, wherein the amino acid sequence of the fusion protein is as shown in SEQ ID NOs: 4, 5, 26, 27, 28.

5. A Fmoc and Boc-modified semaglutide main chain, wherein the position 20 of the semaglutide main chain is a protected lysine, which is a Nε-(tert-butoxycarbonyl)-lysine, and the N-terminus of the semaglutide main chain is a Fmoc-modified histidine.

6. A Boc-modified semaglutide precursor which comprises:
a first semaglutide precursor modified with Boc at position 18, whose amino acid sequence is as shown in SEQ ID NO: 1;
or, a second semaglutide precursor modified with Boc at position 17, whose amino acid sequence is as shown in SEQ ID NO: 2;
or, a third semaglutide precursor modified with Boc at position 16, whose amino acid sequence is as shown in SEQ ID NO: 23;
or, a fourth semaglutide precursor modified with Boc at position 15, whose amino acid sequence is as shown in SEQ ID NO: 24;
or, a fifth semaglutide precursor modified with Boc at position 14, whose amino acid sequence is as shown in SEQ ID NO: 25.

7. A Fmoc-modified semaglutide main chain, wherein the N-terminus of the semaglutide main chain is a Fmoc-modified histidine, and the amino acid sequence of the semaglutide main chain is as shown in SEQ ID NO: 3.

8. An isolated polynucleotide encoding the semaglutide precursor fusion protein of claim 1, the Fmoc and Boc-modified semaglutide main chain of claim 5, the Boc-modified semaglutide precursor of claim 6, or the Fmoc-modified semaglutide main chain of claim 7.

9. A vector comprising the polynucleotide of claim 8.

10. A host cell comprising the vector of claim 9, or in which the chromosome is integrated with exogenous polynucleotide of claim 8, or expressing the semaglutide precursor fusion protein of claim 1, the Fmoc and Boc-modified semaglutide main chain of claim 5, the Boc-modified semaglutide precursor of claim 6, or the Fmoc-modified semaglutide main chain of claim 7.

11. A method for preparing a semaglutide, comprising the steps:
(A) using recombinant bacteria to ferment, to prepare the semaglutide precursor fusion protein of claim 1, and
(B) using the semaglutide precursor fusion protein to prepare the semaglutide.

12. The method according to claim 11, wherein the step (B) further comprising the steps:
(i) digesting the semaglutide precursor fusion protein, thereby obtaining a Boc-modified semaglutide precursor that lacks X amino acids at the N-terminus of the semaglutide main chain, wherein X is an integer of 2-7;
(ii) conjugating a Fmoc complex to the N-terminus of the Boc-modified semaglutide precursor, thereby obtaining a Fmoc and Boc-modified semaglutide main chain;
wherein the Fmoc complex comprises X amino acids at the N-terminus of the semaglutide main chain, and the N-terminal amino acids of the Fmoc complex are modified with Fmoc;
(iii) removing the Boc from the Fmoc and Boc-modified semaglutide main chain, and reacting the same with an semaglutide side chain, thereby obtaining a Fmoc-modified semaglutide; and
(iv) removing the Fmoc from the Fmoc-modified semaglutide, thereby obtaining a Fmoc-removed semaglutide;
(v) removing the OtBu from the side chain of the Fmoc-removed semaglutide, thereby obtaining the semaglutide.

13. The method according to claim 12, wherein in step (i), enterokinase is used for the digestion.

14. The method according to claim 12, wherein in step (ii), the Fmoc complex, DIPEA (N, N-diisopropylethylamine) and DMF (N, N-dimethylformamide) are added to conjugate the Fmoc complex to the N-terminus of the Boc-modified semaglutide precursor,
preferably, the Fmoc complex is an Fmoc complex in the form of an activated ester, formed by activation with HOSu/DCC, HoBt/DIC, TBTU/DIPEA.

15. The method according to claim 12, wherein the step (iii) further comprising the steps:
(a) adding the Fmoc and Boc-modified semaglutide main chain to pre-cooled TFA solution, stirring to remove Boc and obtaining the Boc-removed product;
(b) adding an organic solvent, preferably a mixture of methyl tert-butyl ether/petroleum ether, to the reaction solution of step (a), thereby obtaining a Boc-removed solid product;
(c) mixing the Boc-removed product with the side chain of semaglutide to obtain the Fmoc-modified semaglutide.
